(19) Europäisches Patentamt
European Patent Office
Office européen des brevets

(11) **EP 3 727 030 B1**

(12) **EUROPEAN PATENT SPECIFICATION**

(45) Date of publication and mention
of the grant of the patent:
**16.04.2025 Bulletin 2025/16**

(21) Application number: **18822092.5**

(22) Date of filing: **20.12.2018**

(51) International Patent Classification (IPC):
*A23L 11/30* (2016.01)     *A23J 1/14* (2006.01)
*A23J 3/14* (2006.01)     *A23J 3/16* (2006.01)
*B01J 19/24* (2006.01)     *A61K 36/48* (2006.01)
*A23K 10/12* (2016.01)     *A23L 33/17* (2016.01)
*A23K 10/14* (2016.01)

(52) Cooperative Patent Classification (CPC):
**A23L 11/33; A23J 1/14; A23J 3/14; A23J 3/16;
A23K 10/12; A23K 10/14; A23L 33/17; A61K 36/48**

(86) International application number:
**PCT/EP2018/086306**

(87) International publication number:
**WO 2019/122192 (27.06.2019 Gazette 2019/26)**

(54) **VERTICAL PLUG-FLOW PROCESS FOR BIO-CONVERSION OF BIOMASS INVOLVING ENZYMES**

VERTIKALES PFROPFSTROMVERFAHREN ZUR BIOKONVERSION VON BIOMASSE MITTELS ENZYMEN

PROCÉDÉ DE BIOCONVERSION DE LA BIOMASSE PAR DES ENZYMES À ÉCOULEMENT DE PISTON VERTICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2017 EP 17210107**

(43) Date of publication of application:
**28.10.2020 Bulletin 2020/44**

(73) Proprietor: **Hamlet Protein A/S
8700 Horsens (DK)**

(72) Inventors:
• **DICKOW, Jonatan Ahrens**
**7140 Stouby (DK)**
• **THIRUP, Laila**
**8660 Skanderborg (DK)**
• **ELLEGÅRD, Katrine Hvid**
**8680 Ry (DK)**
• **PETERSEN, Stig Victor**
**8330 Beder (DK)**
• **GELEFF, Svend Andreas**
**6630 Rødding (DK)**

(74) Representative: **Zacco Denmark A/S
Arne Jacobsens Allé 15
2300 Copenhagen S (DK)**

(56) References cited:
EP-A1- 1 264 784          WO-A1-01/54519
WO-A1-98/56260          CN-A- 103 875 982
CN-A- 104 161 168          CN-A- 104 904 991
CN-A- 107 047 928          US-A1- 2002 090 418
US-A1- 2015 141 524          US-B1- 7 452 425

• **MORTEZA KHANAHMADI ET AL: "Continuous solid-state fermentation as affected by substrate flow pattern", CHEMICAL ENGINEERING SCIENCE, vol. 61, no. 8, 6 January 2006 (2006-01-06), GB, pages 2675 - 2687, XP055561991, ISSN: 0009-2509, DOI: 10.1016/ j.ces.2005.11.037**

Remarks:
The file contains technical information submitted after the application was filed and not included in this specification

## Description

### Field of the invention

The present invention relates to a solid substrate, bio-conversion method for the production of a valuable solid transformation product of the substrate wherein the bio-conversion is carried out by the use of one or more suitable enzyme preparations by a continuous plug flow process in a vertical, non-agitated tank where the transport is mediated by gravitational force.

### Background of the invention

There is a need for bio-products that primarily can be used as food or feed or as ingredients in food or feed. The basic constituents in such products are proteins, fats, and carbohydrates. Suitable biomasses for such products are oil bearing crops such as oilseeds, cereals, and legumes. Cereals have a protein content up to 15 % e.g. in wheat, and legumes have a protein content of up to 40 % e.g. in soya beans, based on dry matter. Soya beans are primarily an industrial crop, cultivated for oil and protein. It has a relatively low oil content of the seed, but still soya beans are a large single source of edible oil.

The nutritional quality of protein, measured by its chemical score as essential amino acid composition, and the palatability of protein products are important and essential parameters for nutritional purposes in feed and food products and nutritional supplements. Applications of the protein products in pharma products and cosmetics may sometimes also require high palatability, and/or specific functional properties.

A general problem especially related to pulses and fruits and seeds from legumes which comprise carbohydrates and proteinaceous matter as sources of bio-product (in particular protein products) are the content of indigestible oligosaccharides, such as stachyose and raffinose, causing flatulence and diarrhoea when fermented in the colon.

The approximate average chemical composition of soya bean, taken as an example, measured on moisture-free basis, is 40 % protein; 20 % fat, mostly triglycerides and some phospholipids; 35 % carbohydrate in the form of soluble oligosaccharides (sucrose, raffinose, stachyose, verbascose) and insoluble fibre; and 5 % ash comprising the minerals, in particular potassium, calcium and magnesium.

Proteins from pulses, seeds, cereals, and grasses, including soya bean proteins, such as trypsin inhibitors, allergens and lectins, are known as anti-nutritional factors. They exert specific physiological effects. Trypsin inhibitors impair protein digestion by inactivating trypsin and are considered harmful for the nutritional value of soya bean and are suggested to be responsible for impaired growth in chickens. β-conglycinin is a soy allergen inducing intestinal inflammation and dysfunction.

CN 103 875 982 discloses a solid-state anaerobic fermentation of a feed blend comprising soybean meal with protease at a water content of 32 wt%, at 34 °C for 72 hours.

The object of the present invention is to provide a method for the production of a transformation product of a biomass substrate in a vertical, plug flow bio-conversion process carried out in the presence of one or more suitable enzymes.

Another object is to provide a method, which can be performed in a large and simple reactor design and thereby at low costs.

Yet an object is to provide an efficient method for bio-conversion of biomasses, in particular soya bean or rape seed or mixtures thereof, so as to produce bio-products with desirable properties, such as a high protein content, and/or a modified sugar profile, and/or improved nutritional value, and/or reduced anti-nutritional factors, and/or improved palatability, and/or enhanced organoleptic properties, and/or improved functional properties.

A final object of the invention is to provide an improved method for the production of bio-products comprising a considerable reduced amount of indigestible carbohydrates, and/or anti-nutritional factors.

These objects are fulfilled with the method of the present invention.

### Summary of the invention

Accordingly, in one aspect of the present invention it relates to a method as defined in claim 1 for producing a solid transformation product of a substrate.

The present method for treatment of biomass uses gravitational force to transport/move the biomass during bio-conversion. Although the use of gravity for transportation in general is straightforward, it requires careful selection of reaction conditions for the specific purpose, such as in the case of the present plug-flow process.

Normally, when the water content is increased, an incubation mixture tends to compact, by the reduction of void volume, so that the transportation behaviour is affected negatively. When a certain water content is reached the mixture is compacted to an extent so that the transportation by gravitational force is stopped. The material will stick to the walls of the reactor, and the uniform plug-flow is disrupted resulting in uneven retention time of the biomass.

The solution according to the present invention to the problem connected with transportation by gravitational force of the incubation mixture is to make use of a tank as defined in the claims for incubation wherein the flow of material can be kept so high and uniform that plug-flow conditions are achieved and maintained. The flow rate is regulated by the inlet and outlet means and by the dimensions (width to height ratio) of the tank.

Furthermore, the solution according to the invention must secure balancing of the water content in the incubation mixture so that the water activity on the particle surface is sufficient for the reaction process. This is achieved by keeping the ratio wet bulk density to dry bulk density of the substrate low and within certain limits as defined in claim 1.

More specifically, the present inventors have found that the necessary uniform process can be achieved by using an initial incubation mixture having a water content from 30 % to 70 % by weight, and a ratio of wet bulk density to dry bulk density from 0.60 to 1.45. In combination with the present, vertical design for the plug-flow process it is possible to secure a uniform plug-flow and ensure the same processing time for the incubation mixture. Furthermore, the method of the present invention is conducted without agitation. If the water content exceeds approximately 70 % by weight, the biomass cannot hold the water, and the incubation mixture becomes a slurry having a water phase and a solid phase. These two phases will not flow with the same flow rates, uniform plug flow will not be obtained, and the incubation mixture may stick to the incubator walls. A water content of more than approximately 70 % will result in a ratio of wet bulk density to dry bulk density, exceeding 1.45 that is the upper limit according to the invention.

The vertical design is less expensive in investment than a horizontal design due to its larger capacity in a single production line. It is also less expensive to maintain due to less mechanical movements. The use of a non-agitated tank further contributes to reduced operational costs.

The present method is, in particular, efficient if the substrate of biomass has been pretreated before it is mixed with the enzyme preparation or a combination of enzyme preparations, because the pre-treatment improves the access of the enzymes to the components in the biomass which are to be transformed. The pre-treatment is typically carried out by chemical or physical pre-treatment, e.g. by means of disintegration, milling, flaking, heat treatment, pressure treatment, ultrasonic treatment, hydrothermal treatment, or acid or alkaline treatment.

The method of the invention provides a solid transformation product of the substrate which is a product of the transformation of proteinaceous matter, and/or carbohydrates originating from said biomass.

Such solid transformation products can be used e.g. in a processed food product or as an ingredient in a food or feed product or as an ingredient of a cosmetic or a pharmaceutical product or a nutritional supplement. The solid transformation of the substrate may e.g. be included in a food, feed, cosmetic or pharmaceutical product or a nutritional supplement containing from 1 % to 99% by weight of a solid transformation product.

### *Definitions*

In the context of the current invention, the following terms are meant to comprise the following, unless defined elsewhere in the description.

The terms "about", "around", "approximately", or "~" are meant to indicate e.g. the measuring uncertainty commonly experienced in the art, which can be in the order of magnitude of e.g. +/- 1, 2, 5, 10, 20, or even 50 %.

The term "comprising" is to be interpreted as specifying the presence of the stated part(s), step(s), feature(s), composition(s), chemical(s), or component(s), but does not exclude the presence of one or more additional parts, steps, features, compositions, chemicals or components. E.g., a composition comprising a chemical compound may thus comprise additional chemical compounds, etc.

### Plug-flow process:

In this type of continuous process, the reaction mixture flows through e.g. a tubular or polyhedral reactor with limited back mixing. The flow is a laminar flow where the composition of the reaction mixture changes along the axial direction of the reactor, or a uniform mass flow.

### Biomass:

Comprises biological material, as produced by the photosynthesis and that can be used as raw material in industrial production. In this context, biomass refers to plant matter in the form of seeds, cereals, pulses, e.g. beans and peas, etc., and mixtures thereof, and in particular fruits and seeds of legumes. Furthermore, a biomass comprising pulses is specifically applicable due to the protein content and composition.

The substrate of biomass may be disintegrated by pre-treatment, such as chemical or physical pre-treatment, e.g. by means of disintegration, milling, flaking, heat treatment, pressure treatment, ultrasonic treatment, hydrothermal treatment, or acid or alkaline treatment.

Bio-conversion

Is the process to incubate enzymes on a substrate for a specific purpose, e.g. incubating a protease on a protein to produce peptides or single amino acids.

Solid transformation product of the substrate:

In the present context, solid transformation product of the substrate refers to a product resulting from incubation of the selected biomass with an enzyme preparation, or combination of enzyme preparations, which can convert matter of the substrate to a desirable product, and optionally processing aids.

Bulk density:

Bulk density is a parameter important for the physical behaviour of a biomass which has the form of powder, granules, and the like. The parameter is defined as weight per volume, and may be measured in, e.g., g/ml. It is not an intrinsic property, but can change depending on handling, and can be used as an index of structural changes. The density of a material is determined by placing a fixed volume of the material in a measuring cup and determining the weight or by determining the weight of a measured volume of a material. By this test the following features can be determined:

$$\text{Bulk density (also known as pour density)} = \text{mass/untapped dry volume in g/mL or kg/m}^3;$$

Wet bulk density (also known as total density) = the ratio of the total mass ($M_s$ + $M_l$) to its total volume;

$M_s$ = mass of solids and $M_l$ = mass of liquids.

Thus, in the context of the present invention, "dry bulk density" is the measured bulk density of the biomass without addition of water, viz. the bulk density/pour density. "Wet bulk density" is the bulk density measured after addition of a certain amount of water. Normally, the bulk density is determined in accordance with International Standards ISO 697 and ISO 60, but due to the nature of the substances this was not applicable in the present context. The individual method used is described in the examples.

Oligosaccharides and polysaccharides:

An oligosaccharide is a saccharide polymer containing at least two component monomer sugars. Polysaccharides are saccharide polymers containing many component monomer sugars, also known as complex carbohydrates. Examples include storage polysaccharides such as starch and structural polysaccharides such as cellulose.

Carbohydrates:

Comprise mono-, di-, oligo- and polysaccharides.

Proteinaceous materials:

Comprise organic compounds with a substantial content of proteins made of amino acids arranged in one or more chains. At a chain length of up to approximately 50 amino acids the compound is called a peptide; at higher molecular weight the organic compound is called a polypeptide or a protein.

Fats:

Comprise esters between fatty acids and glycerol. One molecule of glycerol can be esterified to one, two and tree fatty acid molecules resulting in a monoglyceride, a diglyceride or a triglyceride respectively. Usually fats consist of mainly triglycerides and minor amounts of lecithins, sterols, etc. If the fat is liquid at room temperature it is normally called oil. With respect to oils, fats, and related products in this context, reference is made to "Physical and Chemical Characteristics of Oils, Fats and Waxes", AOCS, 1996, as well as "Lipid Glossary 2", F.D. Gunstone, The Oily Press, 2004.

Glycerides:

Comprise mono-, di-, and triglycerides.

Enzymes:

Enzyme(s) is a very large class of protein substances with the ability to act as catalysts. Commonly, and according to the Enzyme Nomenclature Committee Recommendations, they are divided in six classes.
Typical examples in the context of the invention can comprise, but are not limited to, protease(s), peptidase(s), phytase(s), carbohydrase(s), lipase(s), amylase(s), glucosidase(s), amyloglucosidase(s), galactosidase(s), decarboxydase(s), glucanase(s), pectinase(s), cellulase(s), hemicellulase(s), phospholipase(s), transferase(s), and oxidoreductase(s).

Processing aids:

*1. Plant components and organic processing agents*

Some of the functional properties that are important in this context are: Antioxidant, antibacterial action, wetting properties and stimulation of enzyme activity.
The list of plant-based components is huge, but the most important are the following: Rosemary, thyme, oregano, flavonoids, phenolic acids, saponins, and $\alpha$ - and $\beta$ - acids from hops e.g. $\alpha$-lupulic acid for the modulation of soluble carbohydrates.
Furthermore, organic acids e.g. sorbic-, propionic-, lactic-, citric-, and ascorbic acid, and their salts for the adjustment of the pH-value, preservation and chelating properties is part of this group of processing aids.

*2. Inorganic processing agents*

Comprise inorganic compositions that can preserve against bacterial attack during processing, e.g. sodium bisulphite, etc.; anticaking agents, and flow improving agents in the final product, e.g. potassium aluminium silicate, etc.
Comprise inorganic acids e.g. hydrochloric acid or sulphuric acid.

Processed food products:

Comprise dairy products, processed meat products, sweets, desserts, ice cream desserts, canned products, freeze dried meals, dressings, soups, convenience food, bread, cakes, etc.

Processed feed products:

Comprise ready-to-use feed for animals such as piglets, calves, poultry, furred animals, sheep, cats, dogs, fish, and crustaceans, etc.

Pharmaceutical products:

Comprise products, typically in the form of a tablet or in granulated form, containing one or more biologically active ingredients intended for curing and/or alleviating the symptoms of a disease or a condition. Pharmaceutical products furthermore comprise pharmaceutically acceptable excipients and/or carriers. The solid bio products herein disclosed are very well suited for use as a pharmaceutically acceptable ingredient in a tablet or granulate.

Cosmetic products:

Comprise products intended for personal hygiene as well as improved appearance such as conditioners and bath preparations.

**Brief description of drawings**

Figure 1 shows: in lane 1: Marker; Lane 2: Protease treated sample from example 2; Lane 3: Reference sample for both examples 2 and 3; Lane 4: Protease treated sample from example 3.

Figure 2 shows composition of soluble sugars and oligosaccharides. Lane 1: Carbohydrase-treated sample; Lane 2: Reference (soya bean meal).

Figure 3 shows thin layer chromatography showing soluble sugars and oligosaccharides. Lane 1: carbohydrase-treated product; lane 2: reference.

**Detailed description of the invention**

At least 20 % by weight of the biomass comprises carbohydrates and proteinaceous matter originating from soya bean seeds, seed of rape seed, or mixtures thereof.

In a first embodiment of the method of the invention at least 30 % by weight of the biomass, such as at least 40 %, at least 50 %, at least 60 %, at least 70 %, at least 80 %, or at least 90 % by weight, comprises proteinaceous matter originating from optionally defatted soya. The soya may also be dehulled.

In a second embodiment of the method of the invention at least 30 % by weight of the biomass, such as at least 40 %, at least 50 %, at least 60 %, at least 70 %, at least 80 %, or at least 90 % by weight, comprises proteinaceous matter originating from optionally defatted rape seeds.

In a third embodiment of the method of the invention the biomass comprises proteinaceous matter originating from defatted soya flakes in an amount of from 5 % to 95 % by weight in mixture with proteinaceous matter originating from optionally defatted rape seed in an amount of from 95 % to 5 % by weight optionally in further mixture with proteinaceous matter originating from fava beans, peas, sunflower seeds and/or cereals in amounts to make up a total amount of the proteinaceous matter of 100 % by weight.

In any of the embodiments of the invention the biomass comprising proteinaceous matter may further comprise oligosaccharides, and/or polysaccharides, and/or further comprises oils and fats, e.g. from seeds of oil bearing plants.

In any of the embodiments of the invention the solid transformation product of the substrate may be a product of the transformation of proteinaceous matter and/or carbohydrates originating from said biomass, such as a transformation product of pulses, such as soya, pea, lupine, sunflower, and/or cereals, such as wheat, or maize, or from seeds of oil bearing plants, e.g. rape seed.

In any of the embodiments of the invention the substrate, or the substrate after mixing with an enzyme preparation or a combination of enzyme preparations and water, may not comprise any live baker's yeast, and/or it may not comprise any live yeast selected among *Saccharomyces cerevisiae* strains, including spent brewer's yeast and spent distiller's yeast and baker's yeast and spent yeast from wine production, or does not comprise any live yeast; and/or in particular it may not comprise baker's yeast when the enzyme is $\alpha$-galactosidase.

In any of the embodiments of the invention the solid transformation product of the substrate may be a product of the transformation of proteinaceous matter and/or carbohydrates originating from said biomass.

In any of the embodiments of the invention the enzyme preparation or mixture of enzyme preparations comprises one or more enzymes selected from proteases, peptidases, phytases, carbohydrases, such as $\alpha$-galactosidase, amylase, amyloglucosidase, cellulase, pectinase, and hemi-cellulases, e.g. xylanase, mannanase, or glucanase; and lipase, and oxidoreductase.

In any of the embodiments of the invention the dry matter ratio of said substrate of biomass to said enzyme preparation, or said combination of enzyme preparations, may be from 2:1 to 100,000,000:1, such as 1,000:1, 10,000:1, 50,000:1, 100,000:1. 500,000:1, 1,000,000:1, 5,000,000:1, 10,000,000:1, 50,000,000:1, or 100,000,000:1. The skilled person will appreciate that the ratio can be selected in dependence of parameters, such as process conditions, the activity of the enzyme, and the desired product, and the skilled person will be able to optimise the ratio in accordance with these parameters.

In any of the embodiments of the invention water may be added to the substrate in an amount to provide a ratio of wet bulk density to dry bulk density from about 0.60 to 1.45 in the substrate, such as from about 0.65 to about 1.40, e.g. 0.70, 0.75, 0.80, 0.85, 0.90, 0.95, 1.00, 1.10, 1.15, 1.20, 1.25, 1.30, or 1.35.

In any of the embodiments of the invention at least 40 % by weight of the biomass, such as at least 50 %, at least 60 %, at least 70 %, at least 80 % or at least 90 % by weight, may comprise proteinaceous matter originating from optionally defatted rape seeds, whereas water may be added to the substrate in an amount to provide a ratio of wet bulk density to dry bulk density from about 0.65 to about 1.10, such as 0.75, 0.80, 0.85, 0.90, 0.95, 1.00, or 1.05.

In any of the embodiments of the invention one or more processing aids selected from enzymes, plant components, and organic and inorganic processing agents may be added to the substrate before or during incubation.

In any of the embodiments of the invention an $\alpha$-galactosidase preparation may be added to the substrate of biomass and/or to the initial incubation mixture in an amount of from 0.05 to 50 $\alpha$-galactosidase units pr. g. dry matter of substrate of biomass, such as from 0.5 to 25 $\alpha$-galactosidase units pr. g. dry matter of substrate of biomass, e.g. from 1 to 10, from 2 to 8, from 3 to 6, or from 4 to 5 α-galactosidase units pr. g. dry matter of substrate of biomass.

In any of the embodiments of the invention the filling degree of said incubation tank may be kept constant. This will result in a uniform flow.

In any of the embodiments of the invention the incubation can be carried out under anaerobic conditions.

In any of the embodiments of the invention the water content in the incubation mixture may be from 35 % to 70 % by weight, such as 40 %, 45 %, 50 %, 55 %, 60 %, or 65 % by weight.

Thus, the water content in the initial mixture does not exceed 70 % by weight and it may vary from e.g. from 40 % to 65 %, from 45 % to 60 %, from 48 % to 52 %, or 50 % to 55 %, such as 49, 50, 51, 52, 53, or 54 %.

In any of the embodiments of the invention the mixture may be incubated for 1-240 hours at 20-70 °C. The skilled person will know how to optimise the reaction time and the reaction temperature in view of the other reaction conditions, such as the selection of enzyme(s). Thus, the temperature may vary as e.g. 20-65 °C, 25-60 °C, 30-55 °C, 35-50° C, or 40-45 °C; and the reaction time may be selected as e.g. 1 to 180 hours, such as 2 to 150 hours, 3 to 120 hours, 5 to 90 hours, 8 to 72 hours, or 12 to 48 hours, at each and every one of the here mentioned temperature intervals.

In any of the embodiments of the invention the solid transformation product of the substrate may by dried, optionally followed by milling.

In any of the embodiments of the invention the substrate mixture may be incubated at a time and a temperature sufficient to inactivate the enzyme(s), any anti-nutritional factors, and any processing aids, and if used partly or totally, and if desired.

In any of the embodiments of the invention the vertical, non-agitated incubation tank may be closed.

In any of the embodiments of the invention the non-agitated incubation tank can be of a vertical, oblong cylindrical or polyhedral type. The advantage of using this type is that it is space-saving and as it is non-agitated the operating costs and maintenance costs for mixing equipment are avoided.

In any of the embodiments of the invention the area in the upper part of said non-agitated incubation tank may be less than the area in the lower part i.e. the tank is of conical shape. The advantage of this is that the slip effect is increased so that biomasses with a reduced flowability can be used.

In any of the embodiments of the invention the non-agitated incubation tank may have insulating matting or a thermal dimple jacket and means to control the temperature inside the incubation tank.

The solid transformation product of the substrate of the invention may be dried to a water content of not more than 15 %, 13 %, 10 %, 6 %, 4 %, or 2 % by weight and optionally be in milled form.

The solid product of the invention can be a product of the transformation of proteinaceous matter, oligosaccharides, and/or polysaccharides originating from said biomass. The solid transformation product may have reduced content of anti-nutritional factors, such as trypsin inhibitors, antigens, flatulence-producing oligosaccharides, e.g. stachyose and raffinose; phytic acid, and lectin.

The solid product of the invention may comprise at least 40 % proteinaceous matter by weight of dry matter originating from soya.

The solid product of the invention may comprise at least 40 % proteinaceous matter by weight of dry matter originating from rape seed.

The solid product of the invention may comprise proteins in an amount of 30-65 % by weight on dry matter basis originating from plant parts of soya, rape seed, or sun flower, or mixtures thereof.

The solid product of the invention may comprise a total amount of raffinose, stachyose, and verbascose of 3 % by weight or less, such as 2 % or less, 1 % or less, 0.5 % or less, or 0.4 % or less.

Finally, the invention provides a food, feed, cosmetic or pharmaceutical product or a nutritional supplement containing from 1 % to 99 % by weight of a solid transformation product provided according to the invention.

## EXAMPLES

## DENSITY RATIO

### Example 1:

Ratio of wet bulk density / dry bulk density for preferred substrates based on various biomasses

1.1 Biomasses used in the procedure:

*Soya*

The soya used was defatted Soya Bean Meal (SBM).

## EP 3 727 030 B1

*Maize*

The maize used was whole maize, ground on a hammer mill through a 3.5 mm sieve.

*Wheat*

The wheat used was whole wheat, ground on a hammer mill through a 3.5 mm sieve.

*Sunflower*

The sunflower used was defatted Sunflower Seed Meal (SSM).

*Rapeseed*

The rapeseed used was defatted Rape Seed Meal (RSM).

*Fava Beans*

The beans used were whole fava beans.

*Pea protein*

The pea protein used was a pea protein concentrate.

1.2 Description of the procedure:

The amount(s) of biomass and water tabulated in the following was mixed for ten minutes followed by fifty minutes of equilibration in a closed container.
After this the material was poured into a measuring cup of 500 mL and its mass determined by weighing the cup and subtracting the tare of the cup.
The bulk density was calculated as mass/untapped volume in $kg/m^3$.
The dry bulk density used was the measured bulk density of the biomass without addition of water.
The wet bulk density was the bulk density of the biomass with added water.
The ratio was calculated as wet bulk density divided by the dry bulk density.
The moisture content of the biomasses was determined by drying to constant weight.
After addition of water the moisture in the mixture was determined by calculation.

1.3 Results:

The results for 100 % soya and 80 % mixtures with soya are tabulated in the following:

| Soya | Maize | Wheat | Sunflower | Rapeseed | Fava bean | Pea | Water In g | Moisture In % | Bulk Density kg/m $^3$ | Ratio |
|------|-------|-------|-----------|----------|-----------|-----|-----------|---------------|-----------------------|-------|
| 1000 g | | | | | | | 0 | 10.9 | 665 | - |
| 1000 g | | | | | | | 100 | 19.0 | 638 | 0.96 |
| 1000 g | | | | | | | 250 | 28.7 | 500 | 0.75 |
| 1000 g | | | | | | | 450 | 38.6 | 476 | 0.72 |
| 1000 g | | | | | | | 750 | 49.1 | 470 | 0.71 |
| 1000 g | | | | | | | 900 | 53.1 | 572 | 0.86 |
| 1000 g | | | | | | | 1100 | 57.6 | 655 | 0.98 |
| 1000 g | | | | | | | 1400 | 62.9 | 715 | 1.07 |
| 1000 g | | | | | | | 1900 | 69.3 | 889 | 1.34 |

8

(continued)

| Soya | Maize | Wheat | Sunflower | Rapeseed | Fava bean | Pea | Water In g | Moisture In % | Bulk Density kg/m³ | Ratio |
|------|-------|-------|-----------|----------|-----------|-----|-----------|---------------|--------------------|-------|
| | | | | | | | | | | |
| 800 g | 200 g | | | | | | 0 | 11.4 | 703 | - |
| 800 g | 200 g | | | | | | 450 | 38.9 | 617 | 0.88 |
| 800 g | 200 g | | | | | | 900 | 53.4 | 634 | 0.90 |
| 800 g | 200 g | | | | | | 1900 | 69.4 | 1008 | 1.43 |
| | | | | | | | | | | |
| 800 g | | 200 g | | | | | 0 | 11.7 | 694 | |
| 800 g | | 200 g | | | | | 450 | 39.1 | 580 | 0.84 |
| 800 g | | 200 g | | | | | 900 | 53.5 | 623 | 0.90 |
| 800 g | | 200 g | | | | | 1900 | 69.5 | 960 | 1.38 |
| | | | | | | | | | | |
| 800 g | | | 200 g | | | | 0 | 10.4 | 683 | - |
| 800 g | | | 200 g | | | | 450 | 38.2 | 554 | 0.81 |
| 800 g | | | 200 g | | | | 900 | 52.9 | 598 | 0.88 |
| 800 g | | | 200 g | | | | 1900 | 69.1 | 926 | 1.36 |
| | | | | | | | | | | |
| 800 g | | | | 200 g | | | 0 | 11.3 | 711 | |
| 800 g | | | | 200 g | | | 100 | 19.4 | 576 | 0.81 |
| 800 g | | | | 200 g | | | 250 | 29.0 | 514 | 0.72 |
| 800 g | | | | 200 g | | | 450 | 38.8 | 483 | 0.68 |
| 800 g | | | | 200 g | | | 750 | 49.3 | 490 | 0.69 |
| 800 g | | | | 200 g | | | 900 | 53.3 | 597 | 0.84 |
| 800 g | | | | 200 g | | | 1100 | 57.8 | 528 | 0.74 |
| 800 g | | | | 200 g | | | 1900 | 69.4 | 908 | 1.28 |
| | | | | | | | | | | |
| 800 g | | | | | 200 g | | 0 | 11.1 | 691 | - |
| 800 g | | | | | 200 g | | 450 | 38.7 | 569 | 0.82 |
| 800 g | | | | | 200 g | | 900 | 53.2 | 605 | 0.88 |
| 800 g | | | | | 200 g | | 1900 | 69.3 | 941 | 1.36 |
| | | | | | | | | | | |
| 800 g | | | | | | 200 g | 0 | 11.2 | 703 | |
| 800 g | | | | | | 200 g | 450 | 38.7 | 488 | 0.69 |
| 800 g | | | | | | 200 g | 900 | 53.2 | 728 | 1.04 |
| 800 g | | | | | | 200 g | 1900 | 69.4 | 964 | 1.37 |
| | | | | | | | | | | |

The results for 60 % and 40 % of soya mixtures with maize, sunflower and rapeseed as well as 100 % rapeseed are tabulated in the following:

| Soya | Maize | Sunflower | Rapeseed | Water | Moisture In % | Bulk Density kg/m³ | Ratio |
|------|-------|-----------|----------|-------|---------------|--------------------|-------|
| 600 g | 400 g | | | 0 g | 11.8 | 703 | - |
| 600 g | 400 g | | | 250 g | 29.5 | 651 | 0.93 |
| 600 g | 400 g | | | 450 g | 39.2 | 626 | 0.89 |
| 600 g | 400 g | | | 750 g | 49.6 | 631 | 0.90 |
| 600 g | 400 g | | | 900 g | 53.6 | 666 | 0.95 |
| 600 g | 400 g | | | 1100 g | 58.0 | 723 | 1.03 |
| 600 g | 400 g | | | 1400 g | 63.3 | 796 | 1.13 |
| | | | | | | | |
| 600 g | | 400 g | | 0 g | 10.0 | 644 | - |
| 600 g | | 400 g | | 100 g | 18.2 | 530 | 0.82 |
| 600 g | | 400 g | | 250 g | 28.0 | 435 | 0.68 |
| 600 g | | 400 g | | 450 g | 37.9 | 433 | 0.67 |
| 600 g | | 400 g | | 750 g | 48.6 | 436 | 0.68 |
| 600 g | | 400 g | | 900 g | 52.6 | 480 | 0.75 |
| 600 g | | 400 g | | 1100 g | 57.1 | 449 | 0.70 |
| 600 g | | 400 g | | 1400 g | 62.5 | 616 | 0.96 |
| | | | | | | | |
| 600 g | | | 400 g | 0 g | 11.7 | 643 | - |
| 600 g | | | 400 g | 100 g | 19.7 | 560 | 0.82 |
| 600 g | | | 400 g | 250 g | 29.4 | 502 | 0.78 |
| 600 g | | | 400 g | 450 g | 39.1 | 503 | 0.78 |
| 600 g | | | 400 g | 750 g | 49.5 | 492 | 0.77 |
| 600 g | | | 400 g | 900 g | 53.5 | 516 | 0.80 |
| 600 g | | | 400 g | 1100 g | 57.9 | 545 | 0.85 |
| 600 g | | | 400 g | 1400 g | 63.2 | 655 | 1.02 |
| | | | | | | | |
| 400 g | 600 g | | | 0 g | 12.3 | 718 | - |
| 400 g | 600 g | | | 250 g | 29.9 | 636 | 0.89 |
| 400 g | 600 g | | | 450 g | 39.5 | 638 | 0.89 |
| 400 g | 600 g | | | 750 g | 49.9 | 666 | 0.93 |
| 400 g | 600 g | | | 900 g | 53.8 | 721 | 1.00 |
| 400 g | 600 g | | | 1100 g | 58.2 | 802 | 1.12 |
| 400 g | 600 g | | | 1400 g | 63.5 | 988 | 1.38 |
| | | | | | | | |
| 400 g | | 600 g | | 0 g | 9.5 | 654 | - |
| 400 g | | 600 g | | 100 g | 17.7 | 535 | 0.82 |
| 400 g | | 600 g | | 250 g | 27.6 | 422 | 0.65 |
| 400 g | | 600 g | | 450 g | 37.6 | 487 | 0.74 |
| 400 g | | 600 g | | 750 g | 48.3 | 491 | 0.75 |

(continued)

| Soya | Maize | Sunflower | Rapeseed | Water | Moisture In % | Bulk Density kg/m$^3$ | Ratio |
|---|---|---|---|---|---|---|---|
| 400 g | | 600 g | | 900 g | 52.4 | 512 | 0.78 |
| 400 g | | 600 g | | 1100 g | 56.9 | 585 | 0.89 |
| 400 g | | 600 g | | 1400 g | 62.3 | 612 | 0.94 |
| | | | | | | | |
| 400 g | | | 600 g | 0 g | 12.1 | 658 | - |
| 400 g | | | 600 g | 100 g | 20.1 | 556 | 0.84 |
| 400 g | | | 600 g | 250 g | 29.7 | 471 | 0.72 |
| 400 g | | | 600 g | 450 g | 39.4 | 458 | 0.70 |
| 400 g | | | 600 g | 750 g | 49.8 | 486 | 0.74 |
| 400 g | | | 600 g | 900 g | 53.7 | 486 | 0.74 |
| 400 g | | | 600 g | 1100 g | 58.1 | 531 | 0.81 |
| 400 g | | | 600 g | 1400 g | 63.4 | 605 | 0.92 |
| | | | | | | | |
| 0 g | | | 1000 g | 0 g | 12.9 | 616 | - |
| 0 g | | | 1000 g | 100 g | 20.8 | 484 | 0.79 |
| 0 g | | | 1000 g | 250 g | 30.3 | 438 | 0.71 |
| 0 g | | | 1000 g | 450 g | 39.9 | 457 | 0.74 |
| 0 g | | | 1000 g | 750 g | 50.2 | 507 | 0.82 |
| 0 g | | | 1000 g | 900 g | 54.1 | 535 | 0.87 |
| 0 g | | | 1000 g | 1100 g | 58.5 | 585 | 0.95 |
| 0 g | | | 1000 g | 1400 g | 63.7 | 688 | 1.12 |
| | | | | | | | |

**PILOT-SCALE BIO-CONVERSION**

Materials and methods:

*Materials*

Biomasses: Soya Bean Meal (SBM), Soya Flakes, Rape Seed Meal (RSM) and Sunflower Seed Meal (SSM). Water: Normal tap water

Enzymes:

Protease: papain from Enzybel; Ronozyme Pro Act from DSM; Acid protease from Suntaq International Ltd;

$\alpha$-galactosidase: from Bio-Cat (12,500 U/g);

Phytase: Natuphos from BASF

Other carbohydrases: Viscozyme L from Novozymes, Ronozyme VP from Novozymes

**Example 2:**

Bio-conversion with protease on SBM and RSM (50:50 ratio)

2.1 Incubator:

The pilot incubator used was an insulated, cylindrical oblong stainless-steel tube with an effective operating volume of 2 m$^3$ and in- and outlets. Furthermore, the incubator was equipped with a temperature probe at the inlet as well as at the outlet.

2.2 Method:

In a pilot scale vertical reactor with a total volume of 2.0 m$^2$, a continuous inlet amount of 250 kg/h soya bean meal (SBM), 250 kg/h rapeseed meal (RSM), 967 kg/h water at 25°C, 0.5 kg/h protease (papain from Enzybel), and 0.5 kg/h protease (Ronozyme ProAct from DSM) was applied. The ratio wet bulk density/dry bulk density of the incubation mixture was 1.10.
The outlet amount was adjusted to keep a constant level of filling inside the reactor, and the level of filling was set to yield a total processing time of 1.0 hour. Immediately after leaving the vertical reactor, the product was heat treated at 99°C for 15 min followed by air drying.

2.3 Test procedure for product:

The enzyme-treated product and its untreated reference, a 50:50 mixture of SBM and RSM, were analysed for soluble peptides by SDS-PAGE using the following method:
5.0 g of product was suspended in water, pH adjusted to 8.5, and adjusted with water to a total weight of 50.0 g. The suspension was heated to 90°C for 15 min followed by centrifugation at 3000 RCF for 15 min. The supernatant was mixed 1+5 with Laemmli sample buffer + 50 mM DTT (Laemmli, 1970) and heated to 90°C for 15 min. 15 $\mu$L of each sample was loaded onto a TGX Any kD gel and run following the manufacturer's instructions. The gel was stained with colloid coomassie (Kang et al., 2002).

*References:*

Kang D, Gho YS, Suh M, and Kang C, Bull. Korean Chem. Soc., 2002, Vol. 23, No. 11, pp. 1511-1512.

Laemmli UK, Nature, 1970, Vol. 227, pp. 680-685.

2.4 Results:

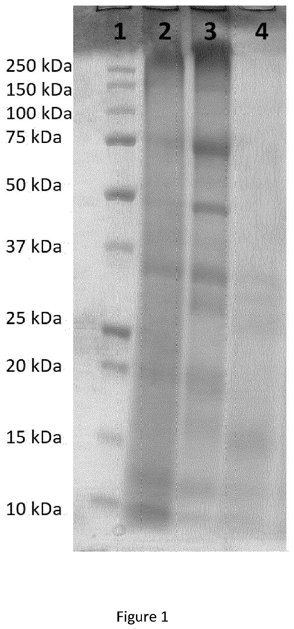

Results of the SDS-PAGE are shown in figure 1, lanes 2 and 3.
It is clear from the SDS-PAGE that most of the distinct protein bands in the untreated SBM/ RSM mixture (lane 3) have been hydrolysed to peptides by the protease treatment in the vertical reactor used according to the invention (lane 2).

**Example 3:**

Bio-conversion with protease on SBM and RSM (50:50 ratio)

3.1 Incubator:

The pilot incubator used was an insulated, cylindrical oblong stainless-steel tube with an effective operating volume of 2 m$^3$ and in- and outlets. Furthermore, the incubator was equipped with a temperature probe at the inlet as well as at the outlet.

3.2 Method:

In a pilot scale vertical reactor with a total volume of 2.0 m$^2$, a continuous inlet amount of 15.6 kg/h soya bean meal (SBM), 15.6 kg/h rapeseed meal (RSM), 24 kg/h water at 40°C, 0.03 kg/h protease (Acid protease from Suntaq International Ltd.), and 0.88 kg/h $H_2SO_4$ was applied. The ratio wet bulk density/dry bulk density of the incubation mixture was 0.75.

The outlet amount was adjusted to keep a constant level of filling inside the reactor, and the level of filling was set to yield a total processing time of 16 hour. Immediately after leaving the vertical reactor, the product was heat treated at 99°C for 15 min followed by air drying.

3.3 Test procedure for product:

The enzyme-treated product and its untreated reference, a 50:50 mixture of SBM and RSM, were analysed for soluble peptides by SDS-PAGE using the following method:

5.0 g of product was suspended in water, pH adjusted to 8.5, and adjusted with water to a total weight of 50.0 g. The suspension was heated to 90°C for 15 min followed by centrifugation at 3000 RCF for 15 min. The supernatant was mixed 1+5 with Laemmli sample buffer + 50 mM DTT (Laemmli, 1970) and heated to 90°C for 15 min. 15 μL of each sample was loaded onto a TGX Any kD gel and run following the manufacturer's instructions. The gel was stained with colloid coomassie (Kang et al., 2002).

*References:*

Kang D, Gho YS, Suh M, and Kang C, Bull. Korean Chem. Soc., 2002, Vol. 23, No. 11, pp. 1511-1512.
Laemmli UK, Nature, 1970, Vol. 227, pp. 680-685.

3.4 Results:

Results of the SDS-PAGE are shown in figure 1, lanes 3 and 4.
It is clear from the SDS-PAGE that almost all of the distinct protein bands in the untreated SBM / RSM mixture (lane 3) have been hydrolysed to peptides by the 16 hour protease treatment in the vertical reactor (lane 4).

**Example 4:**

Bio-conversion with α-galactosidase on soya flakes

This biomass comprises polysaccharides and proteins from pulses

4.1 Incubator

The incubator used was an insulated, cylindrical oblong stainless-steel tube with an internal diameter of 1.55 m and a total height of 4.75 m. In the upper part, there was an array of three rotating paddle type level monitors to regulate the inlet and distribution system to a level at 4.25 m. This gives the incubator an effective operating volume of 8 $m^3$. Furthermore, the incubator was equipped with a temperature probe at the inlet as well as at the outlet.

4.2 Method

A mixture of dehulled, defatted and desolventised soya flakes, sulfuric acid, α-galactosidase, and water at 60°C was prepared continuously in amounts to reach a dry matter content of 50 % by weight in the mixture, a pH of 4.7, and an alpha-galactosidase concentration of 1.1 kg/ton soya. The ratio wet bulk density / dry bulk density of the incubation mixture was 0.73.
The incubator was filled with incubation mixture at a suitable rate per hour. After 16 hours the incubator was filled to operating level and the outlet means were set at a rate to keep the level of filling constant.
An aliquot volume of approx. 30 liters was taken after 18 hours of the test run and incubated at 100 °C with live steam for 25 min.
Subsequently, the wet solid transformation product of the biomass was flash dried and milled.
The overall incubation parameters were the following:

Incubation time - 16 hours

Temperature inlet - 45 °C

Temperature outlet - 45 °C

4.3 Results:

The solid transformation product of the biomass had a total crude protein (N x 6.25) content of 52.6 % and a water content of 5.6 % by weight, which corresponds to a protein of dry matter of 55.5 %. Furthermore, stachyose and raffinose in the dried, solid transformation product were significantly reduced as shown in table 1:

**Table 1**

|  | Soya flakes | Product |
|---|---|---|
| Protein of dry matter | 56 % +/-1% | 55.5 % |
| Stachyose + raffinose | 5-6 % | Absent |

The solid transformation product is highly nutritious and palatable and thus suitable as an ingredient in a number of food and feed products or nutritional supplements. Furthermore, it can be used as an excipient in pharma products and in cosmetics e.g. bath formulations.

**Example 5:**

Bio-conversion with phytase on soya flakes

This biomass comprises polysaccharides and proteins from pulses

5.1 Incubator

The incubator used was an insulated, cylindrical oblong stainless-steel tube with an internal diameter of 1.55 m and a total height of 4.75 m. In the upper part, there was an array of three rotating paddle type level monitors to regulate the inlet and distribution system to a level at 4.25 m. This gives the incubator an effective operating volume of 8 m$^3$. Furthermore, the incubator was equipped with a temperature probe at the inlet as well as at the outlet.

5.2 Method

A mixture of dehulled, defatted and desolventised soya flakes, thermostable phytase, and water at 95 °C was prepared continuously in amounts to reach a dry matter content of 46 % by weight in the mixture, and a phytase concentration of 250 g/ton soya.
The ratio wet bulk density/dry bulk density of the incubation mixture was 0.87.
The incubator was filled with incubation mixture at the rate of 750 liters per hour. After 12 hours the incubator was filled to operating level and the outlet means were set at a rate to keep the level of filling constant.
An aliquot volume of approx. 30 liters was taken after 14 hours of the test run and incubated at 100 °C with live steam for 25 min.
Subsequently, the wet solid transformation product of the biomass was flash dried and milled.
The overall incubation parameters were the following:

Incubation time - 12 hours

Temperature inlet - 67 °C

Temperature outlet - 66 °C

5.3: Results

The solid transformation product of the biomass had a total crude protein (N x 6.25) content of 51.0 % and a water content of 8.0 % by weight, which corresponds to a protein of dry matter of 55.4 %. Furthermore, phytic acid bound phosphor (an anti-nutrient) in the dried, solid transformation product were significantly reduced as shown in table 2:

**Table 2**

|  | Soya flakes | Product |
|---|---|---|
| Protein of dry matter | 55 % +/- 1 % | 55.4 % |
| Phytic acid bound phosphor | 0.42 % | 0.08 % |

The solid transformation product is highly nutritious and palatable and thus suitable as an ingredient in a number of food and feed products or nutritional supplements. Furthermore, it can be used as an excipient in pharma products and in cosmetics e.g. bath formulations.

**Example 6:**

Bio-conversion with carbohydrase on soya bean meal (SBM)

This biomass comprises polysaccharides and proteins from pulses

6.1 Incubator

The incubator was a production scale vertical reactor with a total volume of 96 m$^2$.

6.2 Method

A continuous inlet amount of 1800 kg/h soya bean meal, 2800 kg/h water at 60°C, 6.5 kg/h Viscozyme L from Novozymes, and 2.8 kg/h Depol 679 from Biocatalysts was applied. The ratio wet bulk density/dry bulk density of the incubation mixture was 1.1.

The outlet amount was adjusted to keep a constant level of filling inside the reactor, and the level of filling was set to yield a total processing time of 16 hours. Immediately after leaving the vertical reactor, the product was heat treated at 99°C for 15 min followed by air drying.

6.3 Test procedure for product

Composition of soluble sugars and oligosaccharides was analysed by thin layer chromatography by extracting a watery suspension slurry of 10% DM for 30 min followed by centrifugation for 10 min at 3,000 x g and applying the supernatant onto TLC silica gel 60 plates (Merck). The different components were quantified by comparison to standards of known concentration (Chaplan and Kennedy, 1986).

*References*

Chaplan MF and Kennedy JF. Carbohydrate analysis - practical approach; I RL Press, Oxford, 1986

6.4 Results

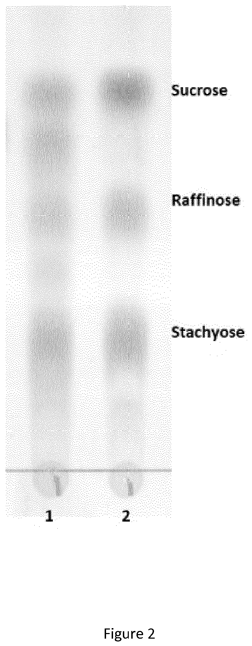

Results of the thin layer chromatography are shown in figure 2. It is evident that the carbohydrase treatment (lane 1) liberates some soluble carbohydrates, visualised by the additional spot between sucrose and raffinose on the thin layer chromatography as well as a general smear throughout the lane compared to the reference (lane 2).

**Example 7:**

Bio-conversion with carbohydrase on soya bean meal (SBM), rapeseed meal (RSM) and wheat

This biomass comprises polysaccharides and proteins from pulses.

7.1 Incubator

The incubator was a laboratory scale plastic container.

7.2 Method

An amount of 60 g soya bean meal, 45 g rapeseed meal, 45 g ground wheat, 180 g water, 3.0 g $H_2SO_4$, 0.525 g Viscozyme L from Novozymes, 0.225 g Depol 679 from Biocatalysts, 0.30 g BAN 480 L from Novozymes, and 0.30 g AMG 300 L from Novozymes was mixed and left to incubate at 37 °C for 16 hours. The ratio wet bulk density/dry bulk density of the incubation mixture was 0.90.
Following incubation, the product was heat treated at 100 °C for 15 min followed by air drying and milling.

7.3 Test procedure for product

The enzyme-treated product and its untreated reference, 60 g soya bean meal + 45 g rapeseed meal + 45 g ground wheat, milled, were analysed for soluble/insoluble non-starch polysaccharides (NSP) using the method of Englyst et al. 1994. Content of carbohydrate content in watery extract was analysed by the phenol sulphuric acid method and composition of soluble sugars and oligosaccharides was analysed by thin layer chromatography by extracting a watery suspension slurry of 10% DM for 30 min followed by centrifugation for 10 min at 3,000 x g and applying the supernatant onto TLC silica gel 60 plates (Merck). The different components were quantified by comparison to standards of known concentration (Chaplan and Kennedy, 1986).

*References*

Chaplan MF and Kennedy JF. Carbohydrate analysis - practical approach; I RL Press, Oxford, 1986

Englyst HN, Quigley ME, and Hudson GJ; Analyst, 1994, Vol 119, pp. 1497-1509.

7.4 Results

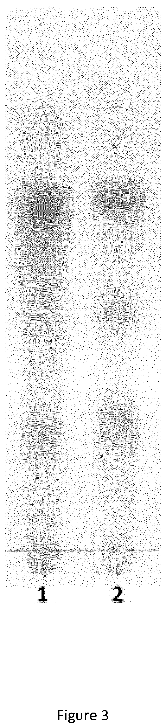

Results of the soluble and insoluble NSP as well as the contents of soluble carbohydrates are shown in table 3. Results of the thin layer chromatography are shown in figure 3.
It is evident that the carbohydrase treatment liberates some soluble carbohydrates, visualised by a smear on the thin layer chromatography, and that the carbohydrase treatment changes the amounts of soluble carbohydrates and NSP present in the product compared to the reference.

**Table 3:** *Contents of soluble/insoluble NSP as well as contents of soluble carbohydrates of enzyme-treated product and its untreated reference, respectively.*

| Product Name | Fraction | Total | |
|---|---|---|---|
| | | g/100g | SD |
| Example 7, product | Soluble NSP | 4.0 | 0.1 |
| | Insoluble NSP | 8.5 | 0.1 |
| | Total NSP | 12.5 | 0.3 |
| | Soluble carbohydrates | 28.7 | 4.8 |
| Example 7, reference | Soluble NSP | 2.8 | 0.2 |
| | Insoluble NSP | 12.0 | 0.2 |
| | Total NSP | 14.8 | 0.5 |
| | Soluble carbohydrates | 15.0 | 4.2 |

**Example 8:**

Bio-conversion with carbohydrase on soya bean meal (SBM) and sunflowe

This biomass comprises polysaccharides and proteins from pulses

8.1 Incubator

The incubator was a pilot scale vertical reactor with a total volume of 2.0 $m^2$.

8.2 Method

A continuous inlet amount of 18.8 kg/h soya bean meal, 12.5 kg/h sunflower meal, 30 kg/h water at 40°C, 0.03 kg/h carbohydrase (Ronozyme VP from Novozymes), and 0.88 kg/h $H_2SO_4$ was applied. The ratio wet bulk density/dry bulk density of the incubation mixture was 0.80.
The outlet amount was adjusted to keep a constant level of filling inside the reactor, and the level of filling was set to yield a total processing time of 16 hour. Immediately after leaving the vertical reactor, the product was heat treated at 99°C for 15 min followed by air drying.

8.3 Test procedure for product

The enzyme-treated product and its untreated reference, 90 g soya bean meal + 60 g sunflower meal, were analysed for soluble/insoluble non-starch polysaccharides (NSP) using the method of Englyst et al. 1994.
*References:* Englyst HN, Quigley ME, and Hudson GJ; Analyst, 1994, Vol 119, pp. 1497-1509.

8.4 Results

The results of the NSP analysis are shown in table 4. It is evident that the processing changes the amounts of the NSP fractions.

Table 4: *Contents of soluble/insoluble NSP as well as contents of soluble carbohydrates of enzyme-treated product and its untreated reference, respectively.*

| Product Name | Fraction | Total | |
|---|---|---|---|
| | | g/100g | SD |
| | Soluble NSP | 3.8 | 1.4 |
| Example 8, product | Insoluble NSP | 12.6 | 1.4 |
| | Total NSP | 16.3 | 0.2 |
| | Soluble NSP | 3.4 | 1.0 |
| Example 8, reference | Insoluble NSP | 14.8 | 1.0 |
| | Total NSP | 18.2 | 0.1 |

**Example 9:**

Large scale bioconversion

Incubator:

The reactor used was a vertical cylinder with an effective height of 7.3 m and a diameter of 4.3 m.
In the top of the vertical reactor, the feed mixture falls on position near the centre of the reactor. For even distribution, a scraper blade or level arm distributes the inlet feed mixture over the perimeter of reactor.
In the bottom of the reactor, the product was extracted by means to achieve a uniform residence time for any particle spread on the top of the reactor.

Testing uniform plug flow

The inlet and outlet means of the reactor were adjusted to achieve an expected residence time of 12 hours. For proving the uniform distribution time, an inert tracer substance was added to the feed mixture. The feed mixture used in the experiment had a natural content of iron of around 143 mg/kg dry matter (= off-set concentration); therefore, iron sulphate ($FeSO_4$) was used as a tracer in a concentration of 1167 mg $FeSO_4$/kg feed mixture dry matter equal to a total iron content of 572 mg Fe/kg total dry matter. At time 0 hours, $FeSO_4$ was added to the feed mixture dosed to the reactor for a period of 60 minutes. Samples were drawn every 20 minutes, dried, and analysed for content of iron, and it was found that the $FeSO_4$ enriched product leaves the reactor 12-13 hours after dosing $FeSO_4$ to the inlet feed mixture, and a maximum concentration of 355 mg/kg Fe was found at 12.5 hours after start.

**Claims**

1.  A method for producing a solid transformation product of a substrate, wherein the solid transformation product is a product of the transformation of proteinaceous matter and/or carbohydrates originating from a biomass, comprising the following steps:

    • preparing a substrate of biomass comprising carbohydrates and proteinaceous matter that originate from soya bean seed, seed of rape seed, or mixtures thereof, wherein at least 20 % by weight of said biomass comprises carbohydrates and proteinaceous matter originating from soya bean seeds, seed of rape seed, or mixtures thereof, optionally in further mixture with carbohydrates and proteinaceous matter originating from seed of fava beans, seed of peas, seed of sunflower, seed of lupine, and/or seed of cereals;
    • mixing said substrate with an enzyme preparation or a combination of enzyme preparations and adding water in an amount which provides an initial incubation mixture having a water content from 30 % to 70 % by weight, and a ratio of wet bulk density to dry bulk density from 0.60 to 1.45, wherein the dry bulk density is the measured bulk density (weight/volume) of the biomass without the added water, and the wet bulk density is the measured bulk density (weight/volume) of the biomass with the added amount of water;
    • incubating said initial incubation mixture for 0.15-240 hours at a temperature of 20-70 °C; and recovering solid transformation product from the incubated mixture;

    further comprising that the incubating step is performed as a uniform, continuous plug-flow process in a vertical, non-agitated incubation tank with inlet means for said mixture and additives and outlet means for said solid transformation product, wherein transport of the biomass within the vertical, non-agitated incubation tank is mediated by gravitational force.

2.  Method according to claim 1, further comprising pre-treatment of said substrate of biomass before it is mixed with said enzyme preparation or said combination of enzyme preparations, such as chemical or physical pre-treatment, e.g. by means of disintegration, milling, flaking, heat treatment, pressure treatment, ultrasonic treatment, hydrothermal treatment, or acid or alkaline treatment.

3.  Method according to any of the claims 1 to 2, wherein at least 30 % by weight of said biomass, such as at least 40 %, at least 50 %, at least 60 %, at least 70 %, at least 80 %, or at least 90 % by weight of said biomass, comprises carbohydrates and proteinaceous matter originating from optionally defatted and/or optionally dehulled soya bean seeds, optionally defatted rape seeds, or mixtures thereof.

4.  Method according to any of the preceding claims wherein said biomass comprises oligosaccharides, and/or polysaccharides, and optionally further comprises oils and fats, e.g. from seeds of oil bearing plants.

5.  Method according to any of the preceding claims wherein said substrate of biomass, or said initial incubation mixture, does not comprise any live yeast, such as any live yeast selected among *Saccharomyces cerevisiae* strains, including spent brewer's yeast and spent distiller's yeast and baker's yeast and spent yeast from wine production.

6.  Method according to any of the preceding claims wherein said solid transformation product of the substrate of biomass is a product of the transformation of proteinaceous matter, or of the transformation of carbohydrates, or of the transformation of proteinaceous matter and carbohydrates originating from pulses, and/or cereals, and/or seeds of oil bearing plants.

7.  Method according to any of the preceding claims, wherein said solid transformation product of the substrate of biomass is a product of the transformation of proteinaceous matter, or of the transformation of carbohydrates, or of the transformation of proteinaceous matter and carbohydrates originating from seeds of soya, pea, lupine, sunflower, wheat, maize, or rape seed.

8.  Method according to any of the preceding claims, wherein said enzyme preparation, or combination of enzyme preparations, comprises one or more enzymes selected from proteases, peptidases, phytases, carbohydrases, lipase, and oxidoreductase, such as one or more carbohydrases selected from α-galactosidase, amylase, amyloglucosidase, pectinase, cellulase, and hemi-cellulases, e.g. xylanase, mannanase, or glucanase.

9.  Method according to any of the preceeding claims, wherein the dry matter ratio of said substrate of biomass to said enzyme preparation, or said combination of enzyme preparations, is from 2:1 to 100,000,000:1, such as 1,000:1, 10,000:1, 50,000:1, 100,000:1. 500,000:1, 1,000,000:1, 5,000,000:1, 10,000,000:1, 50,000,000:1, or 100,000,000:1.

10. Method according to any of the preceeding claims, wherein said initial incubation mixture is incubated for 1 to 180 hours, such as 2 to 150 hours, 3 to 120 hours, 5 to 90 hours, 8 to 72 hours, or 12 to 48 hours.

11. Method according to any of the preceding claims, wherein water is added to said substrate of biomass in an amount which provides an initial incubation mixture having a ratio of wet bulk density to dry bulk density from 0.65 to 1.40, such as 0.70, 0.75, 0.80, 0.85, 0.90, 0.95, 1.00, 1.10, 1.15, 1.20, 1.25, 1.30, or 1.35.

12. Method according to any of the preceding claims, wherein the water content in said initial incubation mixture is from 35 % to 70 % by weight, such as 40%, 45%, 50 %, 55 %, 60 %, or 65 %.

13. Method according to any of the preceding claims, wherein one or more processing aids selected from plant components and organic and inorganic processing agents, such as $\alpha$-galactosidase, are added to said substrate of biomass and/or to said initial incubation mixture.

14. Method according to any of the preceding claims, wherein an $\alpha$-galactosidase preparation is added to the substrate of biomass and/or to the initial incubation mixture in an amount of from 0.05 to 50 $\alpha$-galactosidase units pr. g. dry matter of substrate of biomass, such as from 0.5 to 25 $\alpha$-galactosidase units pr. g. dry matter of substrate of biomass, e.g. from 1 to 10, from 2 to 8, from 3 to 6, or from 4 to 5 $\alpha$-galactosidase units pr. g. dry matter of substrate of biomass.

15. Method according to any of the preceding claims, further comprising that the vertical, non-agitated incubation tank is closed.

16. Method according to any of the preceding claims, wherein said non-agitated incubation tank is of a vertical, oblong cylindrical or polyhedral type, optionally wherein the area in the upper part of said non-agitated incubation tank is less than the area in the lower part i.e. the tank is of conical shape.

17. Method according to any of the preceding claims, wherein said non-agitated incubation tank has insulating matting or a thermal dimple jacket and means to control the temperature in the tank, optionally wherein the filling degree of said incubation tank is kept constant.

**Patentansprüche**

1. Verfahren zum Herstellen eines festen Umwandlungsprodukts eines Substrats, wobei das feste Umwandlungsprodukt ein Produkt der Umwandlung von proteinhaltiger Materie und/oder Kohlenhydraten ist, die ihren Ursprung in Biomasse hat/haben, das die folgenden Schritte umfasst:

   • Zubereiten eines Biomassesubstrats, das Kohlenhydrate und proteinhaltige Materie umfasst, die ihren Ursprung in Sojabohnensaat, Rapssaat oder Gemischen daraus haben, wobei mindestens 20 Gew.-% der Biomasse Kohlenhydrate und proteinhaltige Materie umfasst, die ihren Ursprung in Sojabohnensaat, Rapssaat oder Gemischen daraus haben, optional in einem weiteren Gemisch mit Kohlenhydraten und proteinhaltiger Materie, die ihren Ursprung in Feldbohnensaat, Saat grüner Bohnen, Sonnenblumensaat, Lupinensaat und/oder Getreidesaat haben;
   • Vermischen des Substrats mit einer Enzymzubereitung oder einer Kombination aus Enzymzubereitungen und Hinzufügen von Wasser in einer Menge, die ein anfängliches Inkubationsgemisch bereitstellt, das einen Wassergehalt von 30 bis 70 Gew.-% und ein Verhältnis der Dichte der feuchten Rohmasse zu der Dichte der trockenen Rohmasse von 0,60 bis 1,45 aufweist, wobei die Dichte der trockenen Rohmasse die gemessene Dichte der Rohmasse (Gewicht/Volumen) der Biomasse ohne das hinzugefügte Wasser ist und die Dichte der feuchten Rohmasse die gemessene Dichte der Rohmasse (Gewicht/Volumen) der Biomasse mit der hinzugefügten Menge an Wasser ist;
   • Inkubieren des anfänglichen Inkubationsgemisches für 0,15-240 Stunden bei einer Temperatur von 20-70 °C; und Gewinnen des festen Umwandlungsprodukts aus dem inkubierten Gemisch;

   ferner umfassend, dass der Inkubationsschritt als einheitlicher kontinuierlicher Pfropfenstromprozess in einem vertikalen nicht angeregten Inkubationstank mit einem Einlassmittel für das Gemisch und Zusätze und einem Auslassmittel für das feste Umwandlungsprodukt durchgeführt wird, wobei der Transport der Biomasse innerhalb des vertikalen nicht angeregten Inkubationstanks durch die Schwerkraft verrichtet wird.

2. Verfahren nach Anspruch 1, ferner umfassend eine Vorbehandlung des Biomassesubstrats, bevor es mit der Enzymzubereitung oder der Kombination aus Enzymzubereitungen vermischt wird, wie etwa eine chemische oder physikalische Vorbehandlung, z. B. mittels Desintegration, Mahlen, Abblättern, Wärmebehandlung, Druckbehandlung, Ultraschallbehandlung, hydrothermischer Behandlung oder Säure- oder Alkalibehandlung.

3. Verfahren nach einem der Ansprüche 1 bis 2, wobei mindestens 30 Gew.-% der Biomasse, wie etwa mindestens 40 Gew.-%, mindestens 50 Gew.-%, mindestens 60 Gew.-%, mindestens 70 Gew.-%, mindestens 80 Gew.-% oder mindestens 90 Gew.-% der Biomasse Kohlenhydrate und proteinhaltige Materie umfasst, die ihren Ursprung in optional entfetteten und/oder optional geschälten Sojabohnensamen, optional entfetteten Rapssamen oder Gemischen daraus haben.

4. Verfahren nach einem der vorhergehenden Ansprüche, wobei die Biomasse Oligosaccharide und/oder Polysaccharide umfasst und optional ferner Öle und Fette, z. B aus Samen von ölhaltigen Pflanzen, umfasst.

5. Verfahren nach einem der vorhergehenden Ansprüche, wobei das Biomassesubstrat oder das anfängliche Inkubationsgemisch keine lebendige Hefe, wie etwa eine beliebige lebendige Hefe ausgewählt aus Stämmen von *Saccharomyces cerevisiae,* einschließlich Brauresthefe und Destillierresthefe und Backhefe und Resthefe aus der Weinherstellung, umfasst.

6. Verfahren nach einem der vorhergehenden Ansprüche, wobei das feste Umwandlungsprodukt des Biomassesubstrats ein Produkt der Umwandlung von proteinhaltiger Materie oder der Umwandlung von Kohlenhydraten oder der Umwandlung von proteinhaltiger Materie und Kohlenhydraten ist, die ihren Ursprung in Hülsenfrüchten und/oder Getreiden und/oder Samen von ölhaltigen Pflanzen hat/haben.

7. Verfahren nach einem der vorhergehenden Ansprüche, wobei das feste Umwandlungsprodukt des Biomassesubstrats ein Produkt der Umwandlung von proteinhaltiger Materie oder der Umwandlung von Kohlenhydraten oder der Umwandlung von proteinhaltiger Materie und Kohlenhydraten ist, die ihren Ursprung in Sojasamen, Samen grüner Bohnen, Lupinensamen, Sonnenblumensamen, Weizensamen, Maissamen oder Rapssamen hat/haben.

8. Verfahren nach einem der vorhergehenden Ansprüche, wobei die Enzymzubereitung oder die Kombination aus Enzymzubereitungen ein oder mehrere Enzyme ausgewählt aus Proteasen, Peptidasen, Phytasen, Carbohydrasen, Lipase und Oxidoreductase, wie etwa eine oder mehrere Carbohydrasen ausgewählt aus $\alpha$-Galactosidase, Amylase, Amyloglucosidase, Pektinase, Cellulase und Hemi-Cellulasen, z. B. Xylanase, Mannanase oder Glucanase, umfasst.

9. Verfahren nach einem der vorhergehenden Ansprüche, wobei das Verhältnis von Trockenmasse des Biomassesubstrats zu der Enzymzubereitung oder der Kombination aus Enzymzubereitungen von 2:1 bis 100.000.000:1, wie etwa 1000:1, 10.000:1, 50.000:1, 100.000:1, 500.0000:1, 1.000.000:1, 5.000.000:1, 10.000.000:1, 50.000.000:1 oder 100.000.000:1 beträgt.

10. Verfahren nach einem der vorhergehenden Ansprüche, wobei das anfängliche Inkubationsgemisch für 1 bis 180 Stunden, wie etwa 2 bis 150 Stunden, 3 bis 120 Stunden, 5 bis 90 Stunden, 8 bis 72 Stunden oder 12 bis 48 Stunden, inkubiert wird.

11. Verfahren nach einem der vorhergehenden Ansprüche, wobei dem Biomassesubstrat Wasser in einer Menge hinzugefügt wird, die ein anfängliches Inkubationsgemisch bereitstellt, das ein Verhältnis der Dichte der feuchten Rohmasse zu der Dichte der trockenen Rohmasse von 0,65 bis 1,40, wie etwa 0,70, 0,75, 0,80, 0,85, 0,90, 0,95, 1,00, 1,10, 1,15, 1,20, 1,25, 1,30 oder 1,35 aufweist.

12. Verfahren nach einem der vorhergehenden Ansprüche, wobei der Wassergehalt in dem anfänglichen Inkubationsgemisch von 35 bis 70 Gew.-%, wie etwa 40 Gew.-%, 45 Gew.-%, 50 Gew.-%, 55 Gew.-%, 60 Gew.-% oder 65 Gew.-% beträgt.

13. Verfahren nach einem der vorhergehenden Ansprüche, wobei eine oder mehrere Verarbeitungshilfsmittel ausgewählt aus Pflanzenkomponenten und organischen und anorganischen Verarbeitsmitteln, wie etwa $\alpha$-Galactosidase, dem Biomassesubstrat und/oder dem anfänglichen Inkubationsgemisch hinzugefügt werden.

**14.** Verfahren nach einem der vorhergehenden Ansprüche, wobei dem Biomassesubstrat und/oder dem anfänglichen Inkubationsgemisch eine α-Galactosidasezubereitung in einer Menge von 0,05 bis 50 α-Galactosidaseeinheiten pro Gramm Trockenmasse des Biomassesubstrats, wie etwa von 0,5 bis 25 α-Galactosidaseeinheiten pro Gramm Trockenmasse des Biomassesubstrats, z. B. von 1 bis 10, von 2 bis 8, von 3 bis 6 oder von 4 bis 5 α-Galactosidaseeinheiten pro Gramm Trockenmasse des Biomassesubstrats, hinzugefügt wird.

**15.** Verfahren nach einem der vorhergehenden Ansprüche, ferner umfassend, dass der vertikale nicht angeregte Inkubationstank geschlossen ist.

**16.** Verfahren nach einem der vorhergehenden Ansprüche, wobei der nicht angeregte Inkubationstank vom vertikalen länglichen zylindrischen oder polyedrischen Typ ist und optional wobei der Bereich in dem oberen Teil des nicht angeregten Inkubationstanks kleiner als der Bereich in dem unteren Teil ist, d. h., der Tank eine konische Form aufweist.

**17.** Verfahren nach einem der vorhergehenden Ansprüche, wobei der nicht angeregte Inkubationstank eine Isoliermattierung oder einen thermischen Grübchenmantel und ein Mittel zum Steuern der Temperatur in dem Tank aufweist, optional wobei der Füllstand des Inkubationstanks konstant gehalten wird.

**Revendications**

**1.** Procédé de production d'un produit de transformation solide d'un substrat, dans lequel le produit de transformation solide est un produit de la transformation de matière protéique et/ou de glucides provenant d'une biomasse, comprenant les étapes suivantes :

• la préparation d'un substrat de biomasse comprenant des glucides et de la matière protéique provenant de graines de soja, de graines de colza ou de mélanges de celles-ci, dans lequel au moins 20 % en poids de ladite biomasse comprend des glucides et de la matière protéique provenant de graines de soja, de graines de colza ou de mélanges de celles-ci, éventuellement en mélange supplémentaire avec des glucides et de la matière protéique provenant de graines de féverole, de graines de pois, de graines de tournesol, de graines de lupin et/ou de graines de céréales ;
• le mélange dudit substrat avec une préparation enzymatique ou une combinaison de préparations enzymatiques et l'ajout d'eau en une quantité qui fournit un mélange d'incubation initial ayant une teneur en eau de 30 % à 70 % en poids, et un rapport de la masse volumique apparente humide par rapport à la masse volumique apparente sèche de 0,60 à 1,45, dans lequel la masse volumique apparente sèche est la masse volumique apparente mesurée (poids/volume) de la biomasse sans ajout d'eau, et la masse volumique apparente humide étant la masse volumique apparente mesurée (poids/volume) de la biomasse avec la quantité d'eau ajoutée ;
• l'incubation dudit mélange d'incubation initial pendant 0,15 à 240 heures à une température de 20 à 70 °C ; et la récupération du produit de transformation solide du mélange incubé ;

comprenant en outre que l'étape d'incubation est réalisée sous la forme d'un procédé à écoulement continu uniforme dans une cuve d'incubation verticale non agitée avec des moyens d'entrée pour ledit mélange et des additifs et des moyens de sortie pour ledit produit de transformation solide, dans lequel le transport de la biomasse dans la cuve d'incubation verticale non agitée étant assuré par la force gravitationnelle.

**2.** Procédé selon la revendication 1, comprenant en outre un prétraitement dudit substrat de biomasse avant qu'il ne soit mélangé avec ladite préparation enzymatique ou ladite combinaison de préparations enzymatiques, tel qu'un prétraitement chimique ou physique, par exemple au moyen d'une désintégration, d'un broyage, d'un floconnage, d'un traitement thermique, d'un traitement sous pression, d'un traitement par ultrasons, d'un traitement hydrothermal ou d'un traitement acide ou alcalin.

**3.** Procédé selon l'une quelconque des revendications 1 à 2, dans lequel au moins 30 % en poids de ladite biomasse, tel qu'au moins 40 %, qu'au moins 50 %, qu'au moins 60 %, qu'au moins 70 %, qu'au moins 80 %, ou qu'au moins 90 % en poids de ladite biomasse, comprend des glucides et des matières protéiques provenant de graines de soja éventuellement dégraissées et/ou éventuellement décortiquées, de graines de colza éventuellement dégraissées ou de mélanges de celles-ci.

**4.** Procédé selon l'une quelconque des revendications précédentes, dans lequel ladite biomasse comprend des oligosaccharides et/ou des polysaccharides, et comprend éventuellement, en outre, des huiles et des graisses, provenant par exemple de graines de plantes oléagineuses.

5.  Procédé selon l'une quelconque des revendications précédentes, dans lequel ledit substrat de biomasse, ou ledit mélange d'incubation initial, ne comprend aucune levure vivante, telle que toute levure vivante choisie parmi les souches de *Saccharomyces cerevisiae,* y compris l'excédent de levure de bière et l'excédent de levure de distillerie et la levure de boulangerie et l'excédent de levure de la production de vin.

6.  Procédé selon l'une quelconque des revendications précédentes, dans lequel ledit produit de transformation solide du substrat de biomasse est un produit de la transformation de matière protéique, ou de la transformation de glucides, ou de la transformation de matière protéique et de glucides provenant de légumineuses, et/ou de céréales, et/ou de graines de plantes oléagineuses.

7.  Procédé selon l'une quelconque des revendications précédentes, dans lequel ledit produit de transformation solide du substrat de biomasse est un produit de la transformation de matière protéique, ou de la transformation de glucides, ou de la transformation de matière protéique et de glucides provenant de graines de soja, de pois, de lupin, de tournesol, de blé, de maïs ou de colza.

8.  Procédé selon l'une quelconque des revendications précédentes, dans lequel ladite préparation enzymatique, ou ladite combinaison de préparations enzymatiques, comprend une ou plusieurs enzymes choisies parmi les protéases, les peptidases, les phytases, les carbohydrases, la lipase et l'oxydoréductase, telles qu'une ou plusieurs carbohydrases choisies parmi l'$\alpha$-galactosidase, l'amylase, l'amyloglucosidase, la pectinase, la cellulase et les hémicellulases, par exemple la xylanase, la mannanase ou la glucanase.

9.  Procédé selon l'une quelconque des revendications précédentes, dans lequel le rapport de matière sèche dudit substrat de biomasse par rapport à ladite préparation enzymatique, ou à ladite combinaison de préparations enzymatiques, est de 2 : 1 à 100000000 : 1, tel que 1000 : 1, 10000 : 1, 50000 : 1, 100000 : 1, 500000 : 1, 1000000 : 1, 5000000 : 1, 10000000 : 1, 50000000 : 1 ou 100000000 : 1.

10. Procédé selon l'une quelconque des revendications précédentes, dans lequel ledit mélange d'incubation initial est incubé pendant 1 à 180 heures, tel que 2 à 150 heures, 3 à 120 heures, 5 à 90 heures, 8 à 72 heures ou 12 à 48 heures.

11. Procédé selon l'une quelconque des revendications précédentes, dans lequel de l'eau est ajoutée audit substrat de biomasse en une quantité qui fournit un mélange d'incubation initial ayant un rapport de la masse volumique apparente humide par rapport à la masse volumique apparente sèche de 0,65 à 1,40, tel que 0,70, 0,75, 0,80, 0,85, 0,90, 0,95, 1,00, 1,10, 1,15, 1,20, 1,25, 1,30 ou 1,35.

12. Procédé selon l'une quelconque des revendications précédentes, dans lequel la teneur en eau dudit mélange d'incubation initial est de 35 % à 70 % en poids, tel que 40 %, 45 %, 50 %, 55 %, 60 % ou 65 %.

13. Procédé selon l'une quelconque des revendications précédentes, dans lequel un ou plusieurs auxiliaires de traitement choisis parmi des composants végétaux et des agents de traitement organiques et inorganiques, tel que l'$\alpha$-galactosidase, sont ajoutés audit substrat de biomasse et/ou audit mélange d'incubation initial.

14. Procédé selon l'une quelconque des revendications précédentes, dans lequel une préparation d'$\alpha$-galactosidase est ajoutée au substrat de biomasse et/ou au mélange d'incubation initial en une quantité de 0,05 à 50 unités d'$\alpha$-galactosidase par g de matière sèche de substrat de biomasse, tel que de 0,5 à 25 unités d'$\alpha$-galactosidase par g de matière sèche de substrat de biomasse, par exemple de 1 à 10, de 2 à 8, de 3 à 6 ou de 4 à 5 unités d'$\alpha$-galactosidase par g de matière sèche de substrat de biomasse.

15. Procédé selon l'une quelconque des revendications précédentes, comprenant en outre que le réservoir d'incubation vertical non agité soit fermé.

16. Procédé selon l'une quelconque des revendications précédentes, dans lequel ledit réservoir d'incubation non agité soit de type vertical, cylindrique oblong ou polyédrique, éventuellement dans lequel la superficie dans la partie supérieure dudit réservoir d'incubation non agité soit inférieure à la superficie dans la partie inférieure, c'est-à-dire que le réservoir est de forme conique.

**17.** Procédé selon l'une quelconque des revendications précédentes, dans lequel ledit réservoir d'incubation non agité comporte un revêtement isolant ou une double paroi à alvéoles thermiques et des moyens pour commander la température dans le réservoir, éventuellement dans lequel le degré de remplissage dudit réservoir d'incubation est maintenu constant.

Figure 1

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103875982

**Non-patent literature cited in the description**

- Physical and Chemical Characteristics of Oils, Fats and Waxes. AOCS, 1996
- **F.D. GUNSTONE**. Lipid Glossary 2. The Oily Press, 2004
- **KANG D** ; **GHO YS** ; **SUH M** ; **KANG C**. *Bull. Korean Chem. Soc*, 2002, vol. 23 (11), 1511-1512
- **LAEMMLI UK**. *Nature*, 1970, vol. 227, 680-685
- **CHAPLAN MF** ; **KENNEDY JF**. Carbohydrate analysis - practical approach. I RL Press, 1986
- **ENGLYST HN** ; **QUIGLEY ME** ; **HUDSON GJ**. *Analyst*, 1994, vol. 119, 1497-1509